# EUROPEAN PATENT SPECIFICATION

(11) **EP 1 023 599 B1**
(45) Date of publication and mention of the grant of the patent: **29.05.2002**
(21) Application number: 98944120.9
(22) Date of filing: 25.09.1998
(51) Int. Cl.: G01N 33/50, C12N 5/06

(54) **A MULTICELLULAR IN VITRO ASSAY OF ANGIOGENESIS**
IN VITRO MULTIZELLULAR TEST VON ANGIOGENESIS
DOSAGE MULTICELLULAIRE IN VITRO DE L'ANGIOGENESE

(30) Priority: 01.10.1997 GB 9720987
(43) Date of publication of application: 02.08.2000
(73) Proprietor: BIOCURE LIMITED, Aberdeen AB41 9AA (GB)
(72) Inventor: GRANT, Eileen, Tennant, Aberdeen AB15 5BD (GB); BELL, Graham Thomas, Peebles EH45 8AU (GB); BLOOR, Stephen, Chorley, Lancashire PR6 7QL (GB)
(74) Representative: Ede, Eric
(86) International application number: GB9802908
(87) International publication number: WO9917116

(56) References cited:
- EP-A- 0 358 506
- WO-A-96/40175
- US-A- 4 539 716
- US-A- 4 546 500
- US-A- 5 160 490
- US-A- 5 804 178
- US-A- 5 830 708
- BUSSOLINO F ET AL: "Molecular mechanisms of blood vessel formation" TIBS TRENDS IN BIOCHEMICAL SCIENCES, vol. 22, no. 7, July 1997, page 251-256 XP004081592

## Description

This invention relates to an *in vitro* assay of angiogenesis and in particular a multicellular *in vitro* assay of angiogenesis.

Most populations of differentiated cells in vertebrates are subject to turnover through cell death and renewal. Some fully differentiated cells such as hepatocytes in the liver and endothelial cells lining the blood vessels simply divide to produce daughter cells of the same differentiated type. The proliferation rate of such cells is controlled to maintain the total number of cells. Thus if a large part of the liver is destroyed then the remaining hepatocytes increase their division rate in order to restore the loss.

Endothelial cells form a single cell layer that lines all blood vessels and regulates exchanges between the blood stream and the surrounding tissues. New blood vessels develop from the walls of existing small vessels by the outgrowth of these endothelial cells which have the capacity to form hollow capillary tubes even when isolated in culture. *In vivo,* damaged tissues and some tumours attract a blood supply by secreting factors that stimulate nearby endothelial cells to construct new capillary sprouts. Tumours that fail to attract a blood supply are severely limited in their growth.

The process where new vessels originate as capillaries which sprout from existing small vessels is called angiogenesis. It can therefore be seen that angiogenesis plays a major role in normal tissue development and repair and in the progression of some pathological conditions.

Once the vascular system is fully developed, endothelial cells of blood vessels normally remain quiescent with no new vessel formation. If disease or injury occurs the formation of new blood vessels can proceed normally, as in natural wound healing, or be insufficient, as in chronic dermal ulcers, or there is deregulation of growth and an abnormal increase in vessel density ensues as in tumourogenesis, diabetic retinopathy, psoriasis and inflammation. Inhibition of inappropriate angiogenesis or enhancement of angiogenesis in non-healing wounds is therefore an extremely important target for drug discovery programmes. However, research in this area leading to new drug development has been hindered by the lack of *in vitro* models of angiogenesis.

Angiogenesis is an extremely complex process involving a wide range of growth factors, extracellular matrix molecules, enzymes and various cell types. Such a complexity of relationships has resulted in major difficulties in developing an *in vitro* assay which models the entire *in vivo* process. Angiogenesis can be subdivided into three phases: proliferation, migration and differentiation. Assays exist which model each of these phases separately. Simple *in vitro* tests measure changes in proliferation of a range of cell types and assess migration over basement membrane proteins. Current *in vitro* assay systems which depend on provision of a protein matrix effectively measure the ability of endothelial cells to differentiate.

Assay systems measuring differentiation involve the formation of cord like structures by endothelial cells. All such systems depend on supplying the cells with exogenous basement membrane proteins on which the cells migrate to form tubules. Cell migration occurs over relatively short time periods of 2-16 hours to give a three dimensional structure. In addition to the proteins, many of the systems require the provision of growth factors to produce acceptable tubule formation. The time scale over which tubules are formed provides an excellent test for inhibition of differentiation but is not so useful when testing for enhancement.

The assay systems described above come closest to modelling angiogenesis but none of them combine all three of the stages required for angiogenesis.

There are examples in the prior art of three-dimensional cell culture systems which primarily use an exogenous matrix to mimic the role of base membrane proteins and as such base membrane proteins are not required as the matrix is utilised instead. The matrix allows cells to attach thereto and subsequently grow in more than one layer. The matrices have to have openings of an appropriate size to enable the stromal cells to stretch across the openings. Therefore the correct type of matrix has to be chosen depending on the cells to be cultured. The systems can be used to provide large volumes of differentiated tissue or can be used as model systems for the study of physiological or pathological conditions.

WO 96/39101 discloses the growth of stromal cells on a biodegradable or non-biodegradable three-dimensional support framework, the stromal cells synthesise on said frame work and deposit on the three-dimensional framework a human extracellular matrix as produced in normal human tissue. The extracellular matrix contains various fibre forming proteins interwoven in a hydrated gel composed of a network of glycosaminoglycan chains. The fibre forming protein can be for example collagen and elastin. After several processing steps the processed extracellular matrix can be injected intradermally or subcutaneously into a patient to augment soft tissue, to repair or correct congenital anomalies, acquired defects or cosmetic defects.

On the other hand WO 96/40175 also discloses a stromal cell based three-dimensional cell culture system particularly for use in culturing a variety of different cells and tissues for use in the formation of tubes, tendons, ligaments and corrective structures. The types of materials used for the matrix can be biodegradable such as artificial polymers like collagen.

The three-dimensional tissue culture system disclosed in EP 0 358 506 is based on the fact that growth of stromal **cells** in three dimensions will sustain active proliferation of cells in culture for longer periods than with monolayer systems. It is indicated that such tissues as bone marrow, skin, liver, pancreas etc can all be grown in such a three dimensional system. It is also described that the resulting culture can be used as model systems for the study of physiological or pathological conditions. The matrix can be made from any material or mixture of materials ( for example, treated nylon, gelatin, cellulose and cotton among others) which are generally woven into a mesh to form the three dimensional structure. Those **cells** cultivated on a biodegradable matrix can be implanted in situ into the patient. Those **cells** cultivated on non-biodegradable matrices have to be obtained from the culture and then used.

Lastly US 5 160 490 describes a three-dimensional culture system which can be used to culture a variety of different cells and tissues in vitro for long periods. Cells from a desired tissue are inoculated and grown on a preestablished matrix. The stromal support matrix comprises stromal cells actively growing on a three dimensional matrix. Again as in EP 0 358 506 the matrix can be made from any material or mixture of materials ( for example, treated nylon, gelatin, cellulose and cotton among others) which are generally woven into a mesh to form the three dimensional structure. For subsequent implantation into patients biodegradable matrices should be used such as gelatin. On the other hand where the cultures are to be maintained for long periods of time a nylon mesh type matrix is preferred.

None of these documents makes any mention of use **of** these systems for monitoring the combined stages of angiogenesis. Furthermore the use of a three-dimensional system for use as a model actually makes both visualisation and quantitation extremely difficult. It is therefore quite apparent from the disclosure that the present application relates to a 2-dimensional assay and as such has an advantage over these prior art documents in that it is much more suitable for the purposes of visualisation, and quantitation imaging than the 3-dimensional prior art assays.

It can easily be seen that all the cited documents describe the use of additional exogenous matrices which apparently mimic base membrane proteins to assist with the culture of a variety of different tissues *in vitro* for prolonged periods of time. The whole aim of the present invention was to move away from the use of such systems and provide a simple dual culture system which modelled *in vitro,* the *in vivo* angiogenesis process.

The object of the present invention is to obviate or mitigate the aforesaid disadvantages by providing an *in vitro* assay of angiogenesis which is dependent on all three stages of angiogenesis and can be used to examine both stimulation and inhibition of angiogenesis.

According to one aspect of the present invention there is provided a multicellular *in vitro* assay for modelling the combined stages of angiogenesis namely the proliferation, migration and differentiation stages of cell development, wherein the assay comprises providing a dual culture of endothelial cells together with another cell-type exhibiting interaction therewith to display the combined stages of angiogenesis *in vitro.*

According to an aspect of the invention such an assay relies on use of a dual culture of fibroblasts and endothelial cells and requiring no additional growth factors other than in standard culture medium. It is postulated that the interaction of these cell types is dependent on cell signalling mechanisms therebetween. The non-reliance on additional growth factors is remarkable and unanticipated considering past research on the subject.

According to another aspect of the present invention there is provided a method of screening agents for promoting or inhibiting angiogenesis comprising cultivating a co-culture of endothelial cells together with another cell-type (preferably interstitial cells such as fibroblasts), exhibiting interaction therewith to display the combined stages of angiogenesis, providing a plurality of test containers for same and presenting said agent in controlled amounts to said cultures and observing the containers to monitor angiogenesis. The screening method may be readily automated and angiogenesis may be monitored by known automated counting techniques, image analysis or by spectrographic methods.

Preferably the assay comprises the steps of
(a) setting up growth containers suitable for sustaining dual cell cultures and having a suitable culture medium for sustaining at least growth of endothelial cells therein
(b) seeding a dual culture of human fibroblasts and human endothelial cells to obtain a pre-determined target ratio thereof
(c) incubating same without the provision of any exogenous growth factors
(d) monitoring the progress of the cells from an initial proliferation phase until a confluent monolayer is produced.
(e) changing the culture medium at regular intervals throughout the proliferation, migration and differentiation stages of the cell development.

Preferably the fibroblasts are Human Adult Dermal Fibroblasts and the endothelial cells are Human Umbilical Vein Endothelial Cells (HUVEC).

The cell ratio in the dual culture of Human Adult Dermal Fibroblasts to Human Umbilical Vein Endothelial Cells (HUVEC) is preferably from about 2:1 to 8:1.

Advantageously the culture medium of the dual culture is changed every 48 hours.

According to another aspect of the invention there is provided an assay kit including a vessel provided with culture medium appropriate for sustaining fibroblasts and endothelial cells, and seeded with said cells in a predetermined ratio as a dual culture, wherein the cells are preferably Human Adult Dermal Fibroblasts and Human Umbilical Vein Endothelial Cells (HUVEC) respectively, the viability of the cells in said vessel being monitored for about 24 hours before sending to customers.

Preferably the vessel contains a cell ratio of Human Adult Dermal Fibroblasts to Human Umbilical Vein Endothelial Cells (HUVEC) of about 2:1 to 8:1.

A preferred test kit for use in a multicellular *in vitro* assay comprises a culture vessel seeded with a dual culture of Human Adult Dermal Fibroblasts and Human Umbilical Vein Endothelial Cells (HUVEC) having a cell ratio of about 2:1 to 8:1, said kit further comprising, growth medium capable of sustaining Endothelial cell growth, fixative, blocking buffer, washing buffer, reagents and antibodies for suitable visualisation.

Preferably the reagents for visualisation are those for use in von Willebrand Immunoassay or PECAM-1 Immunoassay.

According to a further aspect of the present invention there is provided a multicellular *in vitro* assay comprising a dual culture of endothelial cells and fibroblasts, preferably Human Adult Dermal Fibroblasts and Human Umbilical Vein Endothelial Cells (HUVEC) and being sustainable in a culture medium, said culture medium capable of sustaining at least endothelial cell growth, the dual culture having been seeded with a cell ratio of about 2:1 to 8:1 of Human Adult Dermal Fibroblasts to Human Umbilical Vein Endothelial Cells (HUVEC) wherein the assay is used to model *in vivo* angiogenesis for use particularly in the likes of drug research or tumour therapy whereby an inhibition of the angiogenesis model by a test drug would indicate its suitability for use in tumour therapy. An enhancement of the angiogenesis model by a test drug would indicate its suitability for use as a wound healing agent.

By virtue of this invention there is provided a multicellular *in vitro* assay which enables examination and modelling for each stage of angiogenesis namely each of the proliferation, migration and differentiation stages of cell development.

The invention will now be described by way of reference to the figures below and also by way of the following examples.
- **Figure 1a-c:**: show the development of the tubules over a period of 1,7 and 14 days
- **Figure 1a:**: Day 1. The darkly stained HUVEC (brown) are clearly visible, positioned on the surface of the fibroblast (blue) monolayer. (x85).
- **Figure 1b:**: Day 7. Thread-like tubules are forming in the confluent fibroblast monolayer. (x34).
- **Figure 1c:**: Day 14. An intricate network of thickened, anastomising vessels has formed, many originating from areas with high fibroblast concentration. (x34)
- **Figure 2a:**: A marked increase in tubule formation on addition of human recombinant vascular endothelial growth factor (VEGF), 10ngml⁻¹. (x34).
- **Figure 2b:**: A marked decrease in tubule formation on addition of anti-human recombinant VEGF neutralising antibody, 10µgml⁻¹ in combination with human recombinant VEGF 10ngml⁻¹ (x34).
- **Figure 3a:**: Incubation with U-87-MG conditioned medium leads not only to the inhibition of tubule formation but also to a massive increase in the number of HUVEC. Note some tubules form at the edges of the large HUVEC "islands". (x34).
- **Figure 3b:**: Incubation with GO-G-CCM conditioned medium effects a marked reduction in HUVEC proliferation. Those cells present, however, have formed small lengths of tubules. (x34)
- **Figure 4:**: Shows an image of tubule formation at day 14, developed by the PECAM-1 immunoassay using BCIP/NBT substrate (alkaline phosphatase compatible). (x34)
- **Figure 5:**: Shows an image of Collagen IV expression in the tubules at day 14. (x85)
- **Figure 6:**: A photographic image of a vertical cross section of the cell layer in the tubule assay at day 14 as seen through the electron microscope (original magnification x7,500).

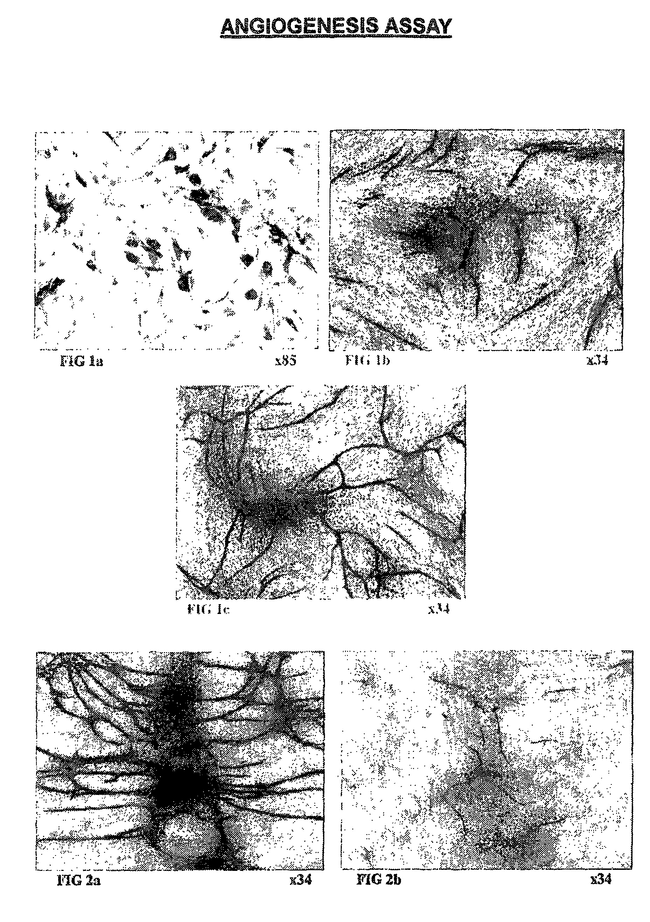

The cultures of Figures 2a and 2b were incubated for 14 days and are therefore directly comparable to Figure 1c.

Visualisation in Figures 1-3 are by von Willebrand factor immunoassay, using DAB substrate, and haematoxylin counterstain.

In accordance with the present invention there is provided an *in vitro* assay for angiogenesis dependent on appropriate cell signalling mechanisms using a dual culture and requiring no additional growth factors. Both stimulation and inhibition of angiogenesis can be demonstrated using this technique.

Furthermore the assay system of the present invention combines all three stages of angiogenesis namely proliferation, migration and differentiation.

The assay system involves co-culture of Human Umbilical Vein Endothelial Cells (HUVEC) with Human Adult Dermal Fibroblasts. Under the conditions provided the cells form a series of anastomosed tubules.

The Human Umbilical Vein Endothelial Cells (HUVEC) are commercially available from suitable outlets and in this case are bought in cryopreserved form. Prior to employment in the tubule assay, the cells are routinely passed and cultured in any suitable commercially available Endothelial Growth Medium, EGM, containing 2% foetal calf serum. The HUVEC are used at passes 2 to 6 in the assay.

The Human Adult Dermal Fibroblasts are cultured in house from skin samples obtained from the local hospitals. Prior to employment in the tubule assay, the cells are routinely passed and cultured in Dulbecco's Modified Eagle's Medium plus 10% foetal calf serum. The fibroblasts are used at passes 6 to 10 in the assay.

The tissue culture treated vessels to be used in the assay are equilibrated by pre-incubation with EGM, plus and minus treatments, for a period of 30 minutes at 37°C, 5% CO₂ humidified atmosphere. Although 12-well and 24-well tissue culture treated plates are normally used others may equally well be employed and the volumes added are lml per well for 12-well plates and 0.5ml per well for 24-well plates. The cells are harvested using any suitable commercially available Trypsin solution for fibroblasts and any suitable commercially available Trypsin-EDTA solution for HUVEC. The cells are resuspended in EGM and counted. Immediately before use, the cells are expressed through a syringe and needle (23G x 1¼) to ensure good dispersal.

The two types of cell are thoroughly mixed at the required densities and seeding ratio (which can be between 2:1 and 8:1, fibroblasts to HUVEC) and added to the plates. In order to ensure an even distribution over the growth surface, the plates are gently agitated in a random fashion. This prevents a pooling of cells in the centre of the wells.

Cell ratios and seeding densities are of paramount importance in this assay. These can vary with each HUVEC line employed and must be established whenever a new line is introduced, to maximise conditions for tubule formation.

The co-cultures are normally incubated over a period of 14 days with complete medium changes approximately every two days. Rudimentary tubule development is evident from around day 4, but, as with all cell types, variations can occur and tubules may form earlier. The whole process can be accelerated to a seven day period or less by increasing the seeding densities whilst maintaining the established ratio. This, however, is not always desirable as the effects of any treatments may be better seen over the long term rather than the short term.

To monitor the progress of the assay, four time points are normally used over a 14 day growth period. This may be altered to suit requirements. At each time point the medium is discarded from the growth vessel and the cells fixed in cold (-20°C) 70% Ethanol for 30 minutes at room temperature. At this point the plate may be washed and stored in phosphate buffered saline at 4°C until completion of the experiment when all the cultures are developed at the same time, or each time point may be processed separately.

To date visualisation of the tubules involves the targeting of one of two cell markers in the HUVEC line by immunoassay. These markers are the glycoprotein von Willebrand factor and the cell adhesion molecule PECAM-1.

Human von Willebrand factor (factor VIII R:Ag) is a multimeric plasma glycoprotein. It mediates platelet adhesion to vessel walls and serves as a carrier and stabiliser for coagulation factor VIII. The factor is synthesised constitutively by endothelial cells. Platelet Endothelial Cellular Adhesion Molecule or PECAM-1 is a 130-KD integral membrane glycoprotein that is the member of the Ig super family and is found constitutively on the surface of endothelial cells, particularly at intercellular junctions. It is also expressed on the surface of platelets and leukocytes.

The immunoassay process involves a two step indirect method where an enzyme-conjugated secondary antibody reacts with an unlabelled primary antibody bound to the cell marker. A substrate solution is added and this reacts with the enzyme complex to produce an insoluble coloured end product. In this way the endothelial tubules are visualised. The co-cultures may be counterstained with haematoxylin nuclear stain. This aids visualisation of the fibroblast monolayer.

Quantitative assessment of the tubules may be achieved by a variety of methods, ranging from manual counting to video imaging and computerised image analysis.

When the HUVEC cells and fibroblasts are incubated together in co-culture without the addition of any exogenous growth factors, but with the complete replacement of the culture medium every two days, the cells initially pass through a proliferative stage which continues until a confluent monolayer is produced. At day 1 the culture consists of a background of fibroblasts with small islands of endothelial cells (Figure 1a). The endothelial cells, continuing to proliferate, enter a migratory phase where they can be seen to move within the fibroblast layer to form thread-like tubular structures at approximately day 7 (Figure 1b). These structures eventually extend and join up to form an intricate network resembling the capillary bed of the chick chorioallantoic membrane at about day 14 (Figure 1c). The "vessels" formed by this process can often be seen to originate from the islands of HUVEC formed during the proliferative phase. High concentrations of fibroblasts are nearly always visible in the area from which the HUVEC have migrated. By day 14 the tubules are wider and thicker with patent lumina which can be visualised with phase-contrast microscopy.

Both the seeding density of the two cell types and the ratio of HUVEC to fibroblasts are extremely critical. The rate at which the HUVEC divide also appears to be critical. By using HUVEC which have widely differing doubling times it can be seen that when the doubling time is short, and therefore the HUVEC are growing very quickly, the outcome tends to be large islands of undifferentiated HUVEC. This would tend to indicate that there is a critical point during the process when intercellular signals between fibroblast and HUVEC initiate the differentiation process.

Experiments were also carried out to show the possibility of using the assay to show the inhibition or stimulation of angiogenesis by a sample under test for example for testing the effects of new drugs.

Vascular endothelial growth factor (VEGF) is a recognised mitogen of endothelial cells and stimulates angiogenesis. Manipulation of the *in vitro* system was confirmed by adding human recombinant VEGF at the start of the experiment and at each medium change. As a result, tubule formation was much enhanced with networks of numerous arcades (Figure 2a).

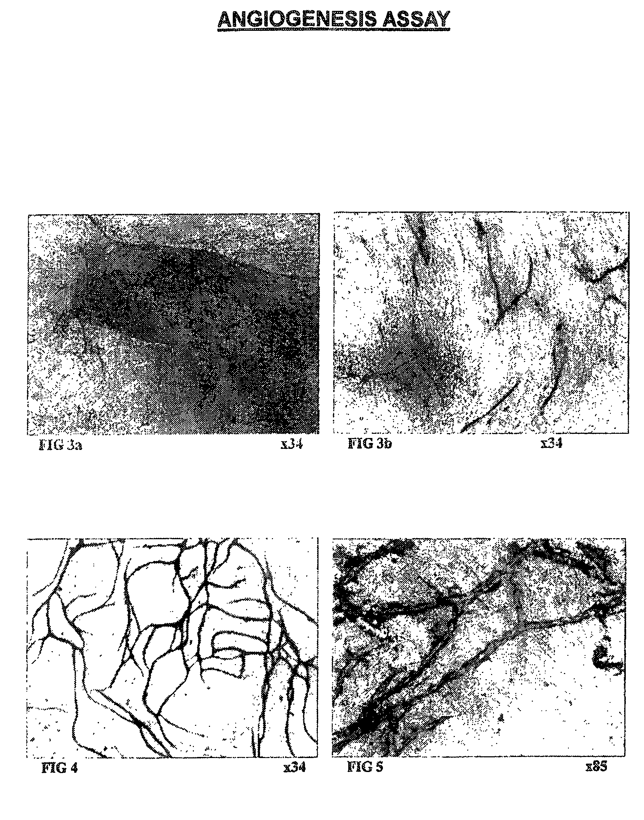

Conversely, when anti-human recombinant VEGF neutralising antibody was added with VEGF, tubule formation was markedly reduced (Figure 2b). The system was also tested using conditioned medium (serum free) from various tumour cell lines including that from U-87-MG (human glioblastoma cell line) which was available from the local hospital and GO-G-CCM (human brain astrocytoma) available from the European Collection Of Animal Cell Cultures (ECACC), for it has been hypothesised that tumour cells can control angiogenesis and in turn favour growth. U-87-MG caused a massive proliferation of HUVEC with very little tubule formation (Figure 3a) whereas GO-G-CCM much reduced HUVEC proliferation and tubule formation (Figure 3b).

These results demonstrate the flexibility of the assay and the response to materials which have different modes of action.

In this way the assay can be used to screen for inhibitors and enhancers of angiogenesis.

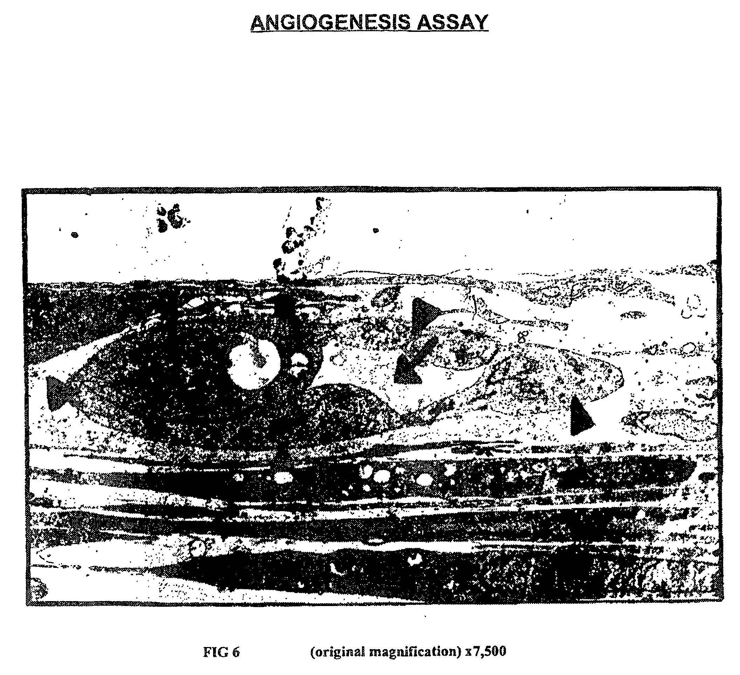

In Figure 5 there is shown an image of Collagen IV expression in the tubules at day 14. *In vivo,* Extra Cellular Matrix (ECM) proteins are laid down by the developing capillaries of neovasculature and this *in vitro* image shows that Collagen IV is selectively expressed by the endothelial cells. This is further evidence that the assay is indeed mimicking the in vivo development of vessels.

Figure 6 shows a photographic image of a vertical cross-section of the cell layer in the tubule assay (day 14) as seen through an electron microscope (original magnification x 7,500). This quite clearly shows a tubule composed of several endothelial cells (shown by arrowheads) encompassing a central lumen (shown by arrow). This presents further proof that the assay is in fact producing tubules with a central "cavity" or lumen.

A control study was also set up to show that not all cells would function if substituted for those currently used in the assay of the present invention. The control study showed HUVEC co-cultured with human umbilical artery smooth muscle cells (HUASMC), cells which form a close association with the endothelial cells *in vivo.* No tubules formed.

It is envisaged that the above invention will give a more accurate study of *in vivo* angiogenesis by using the *in vitro* model for experiments to see the effect of various external factors on same. For example, studying the effect of particular drugs (particularly in the field of tumour therapy) will be greatly assisted by this invention. The assay can be used to determine whether the particular drug under test would inhibit angiogenesis thereby inhibiting tumour growth or whether it would enhance angiogenesis it thereby having applications in wound healing therapy. *In vivo,* damaged tissues and some tumours attract a blood supply by secreting factors that stimulate nearby endothelial cells to construct new capillary sprouts. It can therefore be shown by using this assay whether a particular test drug can prevent the stimulation of the endothelial cells thereby preventing the tumours from attracting a blood supply. Tumours that fail to attract a blood supply are severely limited in their growth. The present invention is also extremely valuable in the study of angiogenesis *per se.*

It is also envisaged that culture vessels will be seeded with viable co-cultures which will be grown up at the preferable cell ratio of between about 2:1 and 8:1, fibroblasts to HUVEC. After approximately 24 hours of co culture the vials would then be suitably packaged in the form of a kit. The kit will also contain the necessary ingredients required to keep the co-culture viable, and also for visualisation of results ( by means of von Willebrand Immunoassay or PECAM-1 Immunoassay).

The preferred test kit for use in the multicellular in vitro assay has a culture vessel seeded with a dual culture of Human Adult Dermal Fibroblasts and Human Umbilical Vein Endothelial Cells (HUVEC) having a cell ratio of about 2:1 to 8:1, a quantity of growth medium capable of sustaining Endothelial cell growth, fixative, blocking buffer, washing buffer, and the reagents and antibodies for suitable visualisation by von Willebrand Immunoassay or PECAM-1 Immunoassay.

Therefore the kit will also contain the following components for use in a von Willebrand Immunoassay:
- (i) a primary antibody -: rabbit anti-human von Willebrand Factor;
- (ii) a secondary antibody -: goat anti-rabbit IgG (whole molecule) Horse radish Peroxidase conjugate; and
- (iii) a substrate -: Horse radish Peroxidase substrate with insoluble end product
and the following components for use in a PECAM-1 Immunoassay:
- (i) a primary antibody -: mouse anti-human PECAM-1;
- (ii) a secondary antibody -: goat anti-mouse IgG (whole molecule) Alkaline Phosphatase conjugate; and
- (iii) a substrate -: Alkaline Phosphatase substrate with insoluble end product.

The completed kits will then be sent out to customers for use in their own research such as angiogenesis research, drug study groups and/or research into wound repair.

## Claims

1. A multicellular *in vitro* assay for modelling the combined stages of angiogenesis namely the proliferation, migration and differentiation stages of cell development, wherein the assay comprises providing a dual culture of endothelial cells together with fibroblasts exhibiting interaction therewith, said cells being provided in the dual culture wherein the ratio of fibroblasts to endothelial cells is from about 2:1 to 8:1, whereby said fibroblasts form a monolayer on which the endothelial cells display the combined stages of angiogenesis such that the respective stages of angiogenesis can be monitored over a predetermined time period.

2. A multicellular *in vitro* assay for modelling the combined stages of angiogenesis according to claim 1 wherein the dual culture further comprises standard culture medium with no additional growth factors or exogenous matrix proteins and a mixture of endothelial cells and fibroblasts which are capable of interaction.

3. A multicellular *in vitro* assay for modelling the combined stages of angiogenesis according to either one of the preceding claims, particularly for screening agents for promoting or inhibiting angiogenesis, comprising providing a plurality of test containers having the dual culture therein, presenting such an agent in selectively controlled amounts to said cultures and observing the containers to monitor the effect on angiogenesis.

4. A multicellular *in vitro* assay for modelling the combined stages of angiogenesis according to claim 3 wherein the screening method is automated and angiogenesis is observed by known automated counting techniques.

5. A multicellular *in vitro* assay for modelling the combined stages of angiogenesis according to claim 3 wherein the angiogenesis is monitored by image analysis.

6. A multicellular *in vitro* assay for modelling the combined stages of angiogenesis according to claim 3 wherein the angiogenesis is monitored by spectrographic methods.

7. A multicellular *in vitro* assay for modelling the combined stages of angiogenesis according to any one of the preceding claims wherein the fibroblasts are Human Adult Dermal Fibroblasts and the endothelial cells are Human Umbilical Vein Endothelial Cells (HUVEC).

8. A multicellular *in vitro* assay for modelling the combined stages of angiogenesis according to any one of the preceding claims wherein the method comprises the steps of
(a) setting up growth containers suitable for sustaining dual cell cultures and having a suitable culture medium for sustaining at least growth of endothelial cells therein,
(b) seeding a dual culture of human fibroblasts and human endothelial cells to obtain a target ratio of 2:1 to 8:1 thereof,
(c) incubating same without the provision of any exogenous growth factors or exogenous matrix proteins,
(d) monitoring the progress of the cells from an initial proliferation phase until a confluent monolayer of human fibroblasts is produced whereon the endothelial cells display the proliferation, migration and differentiation stages of angiogenesis,
(e) changing the culture medium at regular intervals throughout the combined stages angiogenesis,
(f) monitoring the combined stages of angiogenesis or effects thereon of screening agents over a predetermined time period.

9. A multicellular *in vitro* assay for modelling the combined stages of angiogenesis according to claim 8 wherein the fibroblasts are Human Adult Dermal Fibroblasts and the endothelial cells are Human Umbilical Vein Endothelial Cells (HUVEC).

10. A multicellular *in vitro* assay for modelling the combined stages of angiogenesis according to claim 8 or 9 wherein the culture medium of the dual culture is changed every 48 hours.

11. A multicellular *in vitro* assay suitable for modelling *in vivo* angiogenesis according to any one of claims 1-10, for use in research of the angiogenesis process and in therapeutic evaluation of the potential indication of a drug comprising the provision of a dual culture of fibroblasts and endothelial cells in a culture medium in a ratio of about 2:1 to 8:1 thereof, said dual culture being viable and sustainable in said culture medium for a period sufficient to complete the assay, introducing the drug to be evaluated to the dual culture, and observing the effects thereof on cell behaviour, particularly with regard to angiogenesis, whereby an inhibition or enhancement of angiogenesis by said drug indicates a potential for use in the manipulation of diseases and processes which are angiogenesis dependent.

12. A multicellular *in vitro* assay suitable for modelling *in vivo* angiogenesis for use in therapeutic evaluation of the potential indication of a drug according to claim 12 wherein the fibroblasts are Human Adult Dermal Fibroblasts and the endothelial cells are Human Umbilical Vein Endothelial cells (HUVEC).

13. A kit for use in a multicellular *in vitro* assay for modelling the combined stages of angiogenesis wherein the kit comprises a vessel provided with culture medium appropriate for sustaining and allowing interaction between Human Adult Dermal Fibroblasts and Human Umbilical Vein Endothelial Cells (HUVEC), and seeded with said cells in a predetermined ratio of about 2:1 to 8:1 as a dual culture wherein the ratio of fibroblasts to endothelial cells is greater, such that the kit on activation induces the Human Adult Dermal Fibroblasts to form a monolayer on which the Human Umbilical Vein Endothelial Cells (HUVEC) display the combined stages of angiogenesis.

14. A kit according to claim 13 wherein the kit comprises a culture vessel seeded with dual culture, said kit further comprising, growth medium capable of sustaining Endothelial cell growth, fixative blocking buffer, washing buffer, reagents and antibodies for visualisation.

15. A kit according to claim 14 wherein the targeting of suitable markers is used for visualisation.

16. A kit according to claim 14 wherein the reagents for visualisation are those for use in a von Willebrand Immunoassay.

17. A kit according to claim 14 wherein the reagents for visualisation are those for use in a PECAM-1 Immunoassay.

## Patentansprüche

1. Multizellulärer *In-vitro*-Assay für das Modellieren der miteinander kombinierten Phasen der Angiogenese, d. h. der Proliferation, Migration und Differenzierungsphase der Zellentwicklung, **dadurch gekennzeichnet, daß** der Assay das Bereitstellen einer dualen Kultur von Endothelzellen zusammen mit Fibroblasten, die mit den vorgenannten interagieren, umfaßt, wobei die Zellen in der dualen Kultur mit einem Verhältnis von Fibroblasten zu Endothelzellen von etwa 2 : 1 bis 8 : 1 bereitgestellt werden, wobei die Fibroblasten eine Monolayer bilden, auf der die Endothelzellen die miteinander kombinierten Phasen der Angiogenese präsentieren, so daß die betreffenden Phasen der Angiogenese während eines festgelegten Zeitraums überwacht werden können.

2. Multizellulärer *In-vitro*-Assay für das Modellieren der miteinander kombinierten Phasen der Angiogenese nach Anspruch 1, **dadurch gekennzeichnet, daß** die duale Kultur weiterhin ein Standard-Kulturmedium, ohne zusätzliche Wachstumsfaktoren oder exogene Matrixproteine, und eine Mischung aus interaktionsfähigen Endothelzellen und Fibroblasten umfaßt.

3. Multizellulärer *In-vitro*-Assay für das Modellieren der miteinander kombinierten Phasen der Angiogenese nach einem der vorhergehenden Ansprüche, insbesondere für das Screening von Agenzien für die Anregung oder Hemmung der Angiogenese, umfassend das Bereitstellen einer Vielzahl von Testgefäßen, welche die duale Kultur enthalten, das Einbringen eines solchen Agens in selektiv kontrollierten Mengen in die Kulturen und das Beobachten der Gefäße, um die Wirkung auf die Angiogenese zu überwachen.

4. Multizellulärer *In-vitro*-Assay für das Modellieren der miteinander kombinierten Phasen der Angiogenese nach Anspruch 3, **dadurch gekennzeichnet, daß** das Screeningverfahren automatisiert ist und die Angiogenese mittels bekannter automatischer Zählverfahren beobachtet wird.

5. Multizellulärer *In-vitro*-Assay für das Modellieren der miteinander kombinierten Phasen der Angiogenese nach Anspruch 3, **dadurch gekennzeichnet, daß** die Angiogenese mittels spektrographischer Verfahren überwacht wird.

6. Multizellulärer *In-vitro*-Assay für das Modellieren der miteinander kombinierten Phasen der Angiogenese nach Anspruch 3, **dadurch gekennzeichnet, daß** die Angiogenese mittels spektrographischer Verfahren überwacht wird.

7. Multizellulärer *In-vitro*-Assay für das Modellieren der miteinander kombinierten Phasen der Angiogenese nach einem der vorhergehenden Ansprüche, **dadurch gekennzeichnet, daß** die Fibroblasten *Human Adult Dermal Fibroblasts* und die Endothelzellen *Human Umbilical Vein Endothelial Cells* (HUVEC) sind.

8. Multizellulärer *In-vitro*-Assay für das Modellieren der miteinander kombinierten Phasen der Angiogenese nach einem der vorhergehenden Ansprüche, **dadurch gekennzeichnet, daß** das Verfahren folgende Schritte umfaßt:
(a) Vorbereiten von Wachstumsgefäßen, die für die Aufrechterhaltung dualer Zellkulturen geeignet sind und ein geeignetes Kulturmedium aufweisen, das zumindest das Wachsen von Endothelzellen in den Gefäßen aufrecht erhält;
(b) Säen einer dualen Kultur humaner Fibroblasten und humaner Endothelzellen, um ein Soll-Verhältnis derselben von 2 : 1 bis 8 : 1 zu erhalten;
(c) Inkubieren derselben ohne die Bereitstellung exogener Wachstumsfaktoren oder exogener Matrixproteine;
(d) Überwachen der Fortentwicklung der Zellen von einer anfänglichen Proliferationsphase bis zur Bildung einer sich vereinenden Monolayer aus humanen Fibroblasten, auf der die Endothelzellen die Proliferations-, Migrations- und Differenzierungsphase der Angiogenese präsentieren;
(e) regelmäßiges Erneuern des Kulturmediums während der miteinander kombinierten Phasen der Angiogenese;
(f) Überwachen der miteinander kombinierten Phasen der Angiogenese oder der Wirkungen von Screeningagenzien auf dieselbe während eines festgelegten Zeitraums.

9. Multizellulärer *In-vitro*-Assay für das Modellieren der miteinander kombinierten Phasen der Angiogenese nach Anspruch 8, **dadurch gekennzeichnet, daß** die Fibroblasten *Human Adult Dermal Fibroblasts* und die Endothelzellen *Human Umbilical Vein Endothelial Cells* (HUVEC) sind.

10. Multizellulärer *In-vitro*-Assay für das Modellieren der miteinander kombinierten Phasen der Angiogenese nach Anspruch 8 oder 9, **dadurch gekennzeichnet, daß** das Kulturmedium der dualen Kultur alle 48 Stunden erneuert wird.

11. Multizellulärer *In-vitro*-Assay, geeignet für das Modellieren der *In-vivo*-Angiogenese*,* nach einem der Ansprüche 1-10 für die Verwendung in der Forschung bezüglich des Angiogeneseprozesses und in der therapeutischen Bewertung der potentiellen Indikation eines Medikaments, umfassend das Bereitstellen einer dualen Kultur aus Fibroblasten und Endothelzellen in einem Kulturmedium in einem Verhältnis derselben von etwa 2 : 1 bis 8 : 1, wobei die duale Kultur in dem Kulturmedium während eines Zeitraums lebensfähig und aufrecht erhaltbar ist, der ausreicht, um den Assay abzuschließen, das Einbringen des zu bewertenden Medikaments in die duale Kultur und das Beobachten der Wirkungen desselben auf das Verhalten der Zellen, insbesondere im Hinblick auf die Angiogenese, wobei eine Hemmung oder Anregung der Angiogenese durch das Medikament ein Potential für die Verwendung bei der Behandlung von angiogeneseabhängigen Krankheiten und Prozessen anzeigt.

12. Multizellulärer *In-vitro*-Assay*,* geeignet für das Modellieren der *In-vivo*-Angiogenese und für die Verwendung bei der therapeutischen Bewertung der potentiellen Indikation eines Medikaments nach Anspruch 12, **dadurch gekennzeichnet, daß** die Fibroblasten *Human Adult Dermal Fibroblasts* und die Endothelzellen *Human Umbilical Vein Endothelial Cells* (HUVEC) sind.

13. Kit für die Verwendung in einem multizellulären *In-vitro-Assay* für das Modellieren der miteinander kombinierten Phasen der Angiogenese, **dadurch gekennzeichnet, daß** der Kit ein Gefäß umfaßt, das mit einem Kulturmedium versehen ist, das geeignet ist, um *Human Adult Dermal Fibroblasts* und *Human Umbilical Vein Endothelial Cells* (HUVEC) zu erhalten und Interaktion zwischen denselben zu gestatten, und besät mit diesen Zellen in einem festgelegten Verhältnis von etwa 2 : 1 bis 8 : 1 in Form einer dualen Kultur, in der das Verhältnis von Fibroblasten zu Endothelzellen größer ist, so daß der Kit bei Aktivierung bewirkt, daß die *Human Adult Dermal Fibroblasts* eine Monolayer bilden, auf der die *Human Umbilical Vein Endothelial Cells* (HUVEC) die miteinander kombinierten Phasen der Angiogenese präsentieren.

14. Kit nach Anspruch 13, **dadurch gekennzeichnet, daß** der Kit ein Kulturgefäß umfaßt, das mit einer dualen Kultur besät ist, wobei der Kit weiterhin ein Wachstumsmedium, das in der Lage ist, das Wachstum von Endothelzellen aufrecht zu erhalten, Fixativ, Blockungspuffer, Waschpuffer, Reagenzien und Antikörper für die Visualisierung umfaßt.

15. Kit nach Anspruch 14, **dadurch gekennzeichnet, daß** die Visualisierung mit Hilfe des Targetings geeigneter Marker erfolgt.

16. Kit nach Anspruch 14, **dadurch gekennzeichnet, daß** die Reagenzien für die Visualisierung diejenigen sind, die in einem Willebrand-Immunassay verwendet werden.

17. Kit nach Anspruch 14, **dadurch gekennzeichnet, daß** die Reagenzien für die Visualisierung diejenigen sind, die in einem PECAM-1-Immunassay verwendet werden.

## Revendications

1. Essai multicellulaire *in vitro* pour modéliser les étapes d'angiogenèse combinées, à savoir les étapes de prolifération, de migration et de différentiation du développement cellulaire, dans lequel l'essai comprend la fourniture d'une double culture de cellules endothéliales conjointement avec des fibroblastes présentant une interaction avec ces cellules, lesdites cellules étant fournies dans une double culture dans laquelle le rapport fibroblastes/cellules endothéliales est d'environ de 2:1 à 8:1, moyennant quoi lesdits fibroblastes forment une monocouche sur laquelle les cellules endothéliales présentent les étapes d'angiogenèse combinées, de telle sorte que les étapes respectives de l'angiogenèse puissent être surveillées sur une période de temps prédéterminée.

2. Essai multicellulaire *in vitro* pour modéliser les étapes d'angiogenèse combinées selon la revendication 1, dans lequel la double culture comprend en outre un milieu de culture standard sans facteurs de croissance supplémentaires ou protéines matricielles exogènes et un mélange de cellules endothéliales et de fibroblastes qui sont capables d'interaction.

3. Essai multicellulaire *in vitro* pour modéliser les étapes d'angiogenèse combinées selon l'une quelconque des revendications précédentes, en particulier pour sélectionner des agents de sélection favorisant ou inhibant l'angiogenèse, comprenant le fait de fournir une pluralité de récipients à essai contenant la double culture, présenter cet agent dans des quantités sélectivement contrôlées auxdites cultures et observer les récipients pour surveiller l'effet sur l'angiogenèse.

4. Essai multicellulaire *in vitro* pour modéliser les étapes d'angiogenèse combinées selon la revendication 3, dans lequel la méthode de sélection est automatisée et l'angiogenèse est observée selon des techniques de comptage automatique connues.

5. Essai multicellulaire *in vitro* pour modéliser les étapes d'angiogenèse combinées selon la revendication 3, dans lequel l'angiogenèse est surveillée par analyse d'image.

6. Essai multicellulaire *in vitro* pour modéliser les étapes d'angiogenèse combinées selon la revendication 3, dans lequel l'angiogenèse est surveillée par des procédés spectrographiques.

7. Essai multicellulaire *in vitro* pour modéliser les étapes d'angiogenèse combinées selon l'une quelconque des revendications précédentes, dans lequel les fibroblastes sont des fibroblastes dermiques d'adulte humain et les cellules endothéliales sont des cellules endothéliales de veine ombilicale humaine (HUVEC).

8. Essai multicellulaire *in vitro* pour modéliser les étapes d'angiogenèse combinées selon l'une quelconque des revendications précédentes, dans lequel la méthode comprend les étapes consistant à :
(a) préparer des récipients de croissance adaptés pour entretenir des doubles cultures cellulaires et ayant un milieu de culture adapté pour y entretenir au moins la croissance de cellules endothéliales,
(b) ensemencer une double culture de fibroblastes humains et de cellules endothéliales humaines pour obtenir un rapport cible de 2:1 à 8:1 de ceux-ci,
(c) incuber celle-ci sans apporter de facteurs de croissance exogènes ou de protéines matricielles exogènes,
(d) surveiller la progression des cellules depuis une phase de prolifération initiale jusqu'à ce qu'une monocouche confluente de fibroblastes humains soit produite, où les cellules endothéliales présentent les étapes de prolifération, de migration et de différentiation de l'angiogenèse,
(e) changer le milieu de culture à intervalles réguliers tout au long des étapes d'angiogenèse combinées,
(f) surveiller les étapes d'angiogenèse combinées ou les effets sur celle-ci des agents de sélection sur une période de temps prédéterminée.

9. Essai multicellulaire *in vitro* pour modéliser les étapes d'angiogenèse combinées selon la revendication 8, dans lequel les fibroblastes sont des fibroblastes dermiques d'adulte humain et les cellules endothéliales sont des cellules endothéliales de veine ombilicale humaine (HUVEC).

10. Essai multicellulaire in vitro pour modéliser les étapes d'angiogenèse combinées selon la revendication 8 ou 9 dans lequel le milieu de culture de la double culture est changé toutes les 48 heures.

11. Essai multicellulaire *in vitro* adapté pour modéliser une angiogenèse *in vivo* selon l'une quelconque des revendications 1 à 10, destiné à être utilisé dans la recherche du processus d'angiogenèse et dans l'évaluation thérapeutique de l'indication potentielle d'un médicament comprenant la fourniture d'une double culture de fibroblastes et de cellules endothéliales dans un milieu de culture dans un rapport d'environ 2:1 à 8:1 de ceux-ci, ladite double culture étant viable et pouvant être entretenue dans ledit milieu de culture pendant une période suffisamment longue pour réaliser l'essai, en introduisant le médicament à évaluer dans la double culture, et en observant ses effet sur le comportement cellulaire, en particulier en ce qui concerne l'angiogenèse, moyennant quoi une inhibition ou une amélioration de l'angiogenèse par ledit médicament indique un potentiel d'utilisation dans la manipulation de maladies et de processus qui dépendent de l'angiogenèse.

12. Essai multicellulaire *in vitro* adapté pour modéliser une angiogenèse *in vivo* destiné à être utilisé dans l'évaluation thérapeutique de l'indication potentielle d'un médicament selon la revendication 12, dans lequel les fibroblastes sont des fibroblastes dermiques d'adulte humain et les cellules endothéliales sont des cellules endothéliales de veine ombilicale humaine (HUVEC).

13. Trousse destinée à être utilisée dans un essai multicellulaire *in vitro* pour modéliser les étapes d'angiogenèse combinées, dans laquelle la trousse comprend un récipient contenant un milieu de culture approprié pour entretenir et permettre une interaction entre des fibroblastes dermiques d'adulte humain et des cellules endothéliales de veine ombilicale humaine (HUVEC), et ensemencé avec lesdites cellules dans un rapport prédéterminé d'environ 2:1 à 8:1 comme une double culture, dans laquelle le rapport fibroblastes/cellules endothéliales est supérieur, de telle sorte que la trousse, lorsqu'elle est activée, induit les fibroblastes dermiques d'adulte humain à former une monocouche sur laquelle les cellules endothéliales de veine ombilicale humaine (HUVEC) présentent les étapes d'angiogenèse combinées.

14. Trousse selon la revendication 13, dans laquelle la trousse comprend un récipient de culture ensemencé avec une double culture, ladite trousse comprenant en outre un milieu de croissance capable d'entretenir la croissance des cellules endothéliales, un tampon de blocage fixatif, un tampon de lavage, des réactifs et des anticorps à des fins de visualisation.

15. Trousse selon la revendication 14, dans laquelle le ciblage de marqueurs adaptés est utilisé à des fins de visualisation.

16. Trousse selon la revendication 14, dans laquelle les réactifs pour la visualisation sont ceux destinés à être utilisés dans un essai immunologique de Willebrand.

17. Trousse selon la revendication 14, dans laquelle les réactifs pour la visualisation sont ceux destinés à être utilisés dans un essai immunologique PECAM-1.
